# FASCICULE DE BREVET EUROPEEN

(11) **EP 1 127 042 B1**
(45) Date de publication et mention de la délivrance du brevet: **23.07.2003**
(21) Numéro de dépôt: 99950883.1
(22) Date de dépôt: 29.10.1999
(51) Int. Cl.: C07C 51/12, C07C 51/353, C07C 67/36, C07C 53/08, C07C 69/14

(54) **PREPARATION D'ACIDE ACETIQUE ET/OU D'ACETATE DE METHYLE EN PRESENCE D'IRIDIUM ET DE PLATINE**
HERSTELLUNG VON ESSIGSÄURE UND/ODER METHYLACETAT IN GEGENWART VON IRIDIUM UND PLATIN
METHOD FOR PREPARING ACETIC ACID AND/OR METHYL ACETATE IN THE PRESENCE OF IRIDIUM AND PLATINUM

(30) Priorité: 05.11.1998 FR 9813954
(43) Date de publication de la demande: 29.08.2001
(73) Titulaire: ACETEX CHIMIE, 92205 Neuilly sur Seine Cedex (FR)
(72) Inventeur: LE BERRE, Carole, F-31120 LA CROIX-FALGARDE (FR); KALCK, Philippe, F-31320 Auzeville-Tolosane (FR); SERP, Philippe, F-31400 Toulouse (FR); LAYEILLON, Lise, F-64170 AUDEJOS (FR); THIEBAUT, Daniel, F-64230 Lescar (FR)
(74) Mandataire: Giraud, Françoise
(86) Numéro de dépôt international: FR9902652
(87) Numéro de publication internationale: WO00027785

(56) Documents cités:
- WO-A-97/35828
- WO-A-98/33759
- FR-A- 2 750 985
- GB-A- 1 234 641

## Description

La présente invention concerne un procédé de préparation en phase liquide d'acide acétique et/ou d'acétate de méthyle en présence d'un catalyseur homogène comprenant de l'iridium et du platine.

Le procédé de l'invention permet d'obtenir une productivité augmentée en acide acétique ainsi qu'une stabilité améliorée du système catalytique employé.

Diverses voies d'accès à l'acide acétique sont connues et exploitées industriellement. Parmi celles-ci figure la réaction de carbonylation du méthanol mise en oeuvre en phase liquide, sous pression de monoxyde de carbone qui est l'un des réactifs, en présence d'un système catalytique homogène. Une autre voie d'accès à l'acide acétique consiste à effectuer l'isomérisation du formiate de méthyle. Cette réaction est, elle-même, généralement réalisée en présence d'un système catalytique en phase homogène. Enfin, selon un autre procédé, on réalise simultanément la carbonylation du méthanol et l'isomérisation du formiate de méthyle.

Plus précisément, le procédé de carbonylation au rhodium est un procédé connu, exploité industriellement et ayant fait l'objet de nombreux articles et brevets comme, par exemple, les brevets américains US 3,769,329 et US 3,813,428.

Les brevets européens EP 618 183 et EP 618 184 ainsi que les brevets européens EP 785 919 et EP 759 022 décrivent un procédé de carbonylation en présence d'un système catalytique à base d'iridium et, le cas échéant, contenant en plus du rhodium.

Un procédé de carbonylation à l'iridium et au ruthénium, actuellement exploité industriellement, est décrit dans le brevet européen EP 643 034.

Plus récemment, une nouvelle voie d'accès constituée par la réaction d'isomérisation du formiate de méthyle en présence d'iridium a été proposée dans le brevet français FR 2 746 794 et la demande internationale WO 97/35829.

Parallèlement, il a été proposé dans le brevet FR 2 746 795 et la demande internationale WO 97/35828 un procédé de préparation d'acide acétique et/ou d'acétate de méthyle mettant en oeuvre simultanément la réaction d'isomérisation du formiate de méthyle et la réaction de carbonylation du méthanol.

Ces différents procédés de production d'acide acétique sont en général mis en oeuvre en continu dans des installations comprenant essentiellement trois zones. La première correspond à la zone de réaction proprement dite, comprenant un réacteur sous pression dans lequel la carbonylation et/ou l'isomérisation sont effectuées. La seconde est constituée par une zone de séparation de l'acide formé. Cette opération est réalisée par vaporisation partielle du mélange réactionnel dans un appareil appelé flash où la pression est maintenue plus faible que dans le réacteur. La partie vaporisée est ensuite envoyée dans une troisième zone où l'acide acétique produit est purifié. Celle-ci comprend diverses colonnes de distillation dans lesquelles l'acide acétique produit est séparé de l'eau, des réactifs et des sous-produits. La partie du mélange restée sous forme liquide en sortie de la zone de vaporisation et comprenant notamment le catalyseur est recyclée au réacteur.

Le procédé selon l'invention a pour objet un perfectionnement des procédés décrits ci-dessus, qui met en oeuvre un système catalytique en phase homogène comprenant de l'iridium et du platine.

Il a en effet été maintenant trouvé que l'ajout de platine à un système catalytique à base d'iridium ou d'iridium et de rhodium permettait d'augmenter d'une façon totalement inattendue, la vitesse de production de l'acide. En d'autres termes, le procédé selon l'invention permet d'atteindre une vitesse de réaction supérieure ou égale à celle obtenue, dans les mêmes conditions, avec un système catalytique ne mettant en jeu que de l'iridium ou un mélange iridium/rhodium, alors que le nombre de moles total en métaux utilisés dans le procédé de l'invention demeure inchangé.

Outre l'augmentation de la productivité, le fait d'obtenir des vitesses comparables en mettant en jeu un nombre de moles de catalyseur inférieur, constitue un avantage supplémentaire d'ordre économique : celui de diminuer les coûts en catalyseur.

Il a été, en outre, mis en évidence que le platine accentue notablement la stabilité de l'iridium, même à faible teneur en eau.

Le procédé selon l'invention est mis en oeuvre en phase liquide. Par conséquent le système catalytique mis en oeuvre se présente sous forme soluble dans le milieu réactionnel.

Le système catalytique convenable à la réalisation de l'invention est à base d'au moins un composé de l'iridium, seul ou en présence de rhodium, d'au moins un promoteur halogéné et comprend en outre au moins un dérivé de platine.

Ainsi, selon une de ses caractéristiques essentielles, l'invention concerne un procédé de préparation d'acide acétique et/ou d'acétate de méthyle en phase liquide par carbonylation du méthanol et/ou isomérisation du formiate de méthyle en présence d'eau, d'un solvant, d'un système catalytique homogène comprenant de l'iridium et un promoteur halogéné et de monoxyde de carbone, caractérisé en ce que ledit système catalytique comprend en outre du platine.

L'invention consiste donc en un perfectionnement aux procédés de préparation d'acide acétique par isomérisation, par carbonylation ou encore par un procédé mixte d'isomérisation et carbonylation mettant en oeuvre un système catalytique à base d'iridium soluble et un promoteur halogéné et selon lequel on ajoute audit système catalytique, du platine sous une forme soluble dans le milieu.

Selon une première variante du procédé de l'invention, on réalise la carbonylation du méthanol en maintenant pendant la réaction une pression partielle de monoxyde de carbone comprise entre 0,1.10⁵ Pa et 200.10⁵ Pa.

Selon une seconde variante du procédé, on réalise l'isomérisation du formiate de méthyle en maintenant une pression partielle de monoxyde de carbone comprise entre 0,1.10⁵ Pa et 25.10⁵ Pa pendant la réaction. Les conditions préférées de la mise en oeuvre d'un tel procédé peuvent être directement obtenues par l'homme du métier en se référant à la demande internationale WO 97/35829 citée précédemment, mais en ajoutant le platine à l'iridium.

Selon une troisième variante du procédé, on réalise simultanément une réaction de carbonylation du méthanol et d'isomérisation du formiate de méthyle en maintenant une pression partielle de monoxyde de carbone comprise entre 0,1.10⁵ Pa et 25.10⁵ Pa pendant la réaction. Les conditions préférées de la mise en oeuvre d'un tel procédé peuvent être directement obtenues par l'homme du métier en se référant à la demande internationale WO 97/35828 citée précédemment, mais en ajoutant le platine à l'iridium.

Dans le système catalytique utilisé dans les trois variantes précédentes, on pourra avantageusement remplacer l'iridium par une combinaison iridium + rhodium.

Lorsque l'on aura recours à un système catalytique contenant du rhodium, le rapport atomique du rhodium à l'iridium pourra varier dans une large gamme comprise entre 0,01 et 99.

Dans toutes ces variantes, le platine sera introduit en quantité suffisante et dans des proportions adéquates par rapport à l'iridium dans le milieu réactionnel. Des essais réalisés par les inventeurs de la présente invention ont en effet montré que les quantités et proportions optimales de platine étaient intimement liées à la forme sous laquelle le platine était introduit dans le milieu réactionnel.

Précisément, tous les composés du platine solubles ou pouvant être solubilisés dans le milieu réactionnel, dans les conditions de l'invention, peuvent être utilisés.

A titre d'exemple et sans intention de se limiter, conviennent notamment à la mise en oeuvre de l'invention :
- des composés ci-après désignés par "composés simples" du platine tel que, le platine à l'état métallique, ses sels et ses oxydes.
- des complexes de coordination de ce métal.

Les composés sous forme de complexes s'avèrent les composés préférés selon l'invention.

En tant que sels, on utilise, de préférence, les halogénures de platine. L'halogène est plus particulièrement choisi parmi le chlore, le brome ou l'iode, ce dernier étant préféré.

Ainsi, les composés comme PtI₂, PtBr₂, PtCl₂, PtCl₄.xH₂O, H₂PtCl₆.xH₂O, Na₂PtCl₄.xH₂O, Na₂PtCl₆.xH₂O peuvent être utilisés dans le procédé selon l'invention.

Les oxydes choisis parmi PtO, PtO₂, PtO₂.xH₂O peuvent de même être convenablement mis en oeuvre dans le procédé selon l'invention.

En ce qui concerne les complexes de coordination solubles du platine, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles du platine dont les ligands sont choisis parmi des composés organo phosphorés ou organo azotés, par exemple.

En tant que complexes de coordination connus de l'homme du métier, convenant particulièrement à la mise en oeuvre de l'invention, on peut citer sans intention de se limiter les composés suivants : PtI₂(CO)₂, [PtI₂(CO)]₂, [Pt₃(CO)₆]²⁻[Q⁺]₂, [Pt₆(CO)₁₂]²⁻[Q⁺]₂ ; formules dans lesquelles Q peut représenter notamment l'hydrogène, les groupes NR₄, PR₄, avec R choisi parmi l'hydrogène et/ou un radical hydrocarboné, le tétraiodo dicarbonyle diplatine [PtI₂(CO)]₂ étant préféré.

Selon une première variante de l'invention, lorsque le platine est introduit sous forme d'un composé simple- platine à l'état métallique, sels, oxydes -, on maintiendra de préférence une teneur en platine d'au moins 4 mmoles/l de milieu réactionnel et un rapport atomique de l'iridium au platine compris entre 2 et 5.

Selon une deuxième variante particulièrement préférée de l'invention, lorsque le platine est introduit sous forme d'un complexe de coordination de ce métal, avec des ligands choisis parmi le monoxyde de carbone ou une combinaison monoxyde de carbone/halogène ou des composés organoazotés ou organophosphorés, on maintiendra de préférence une teneur en platine d'au moins 1 mnole/l de milieu réactionnel et un rapport atomique de l'iridium au platine compris entre 1 et 5.

Le rapport atomique de l'iridium ou, le cas échéant, lorsque le système catalytique comprend en outre du rhodium, de l'ensemble (iridium + rhodium) au platine est compris entre 2 et 5 avec le platine utilisé sous forme de composés simples tels que définis précédemment et entre 1 et 5 quand le platine est introduit sous forme de complexes de coordination. La teneur en platine sera de préférence d'au moins 4 mmoles/l de milieu réactionnel dans le cas des composés simples du platine et d'au moins 1 mmole/l dans le cas des complexes de coordination.

Généralement, la concentration dans le milieu réactionnel en iridium ou le cas échéant, en (iridium + rhodium) est comprise entre 0,1 et 100 mmol/l, de préférence entre 1 et 20 mmol/l.

Tous les composés à base de rhodium et d'iridium, utilisés habituellement dans les réactions de carbonylation et/ou d'isomérisation, peuvent être mis en oeuvre dans le procédé selon l'invention.

Tous les composés de l'iridium solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention l'iridium à l'état métallique, les sels de ce métal, les oxydes ou encore les complexes de coordination.

En tant que sels d'iridium, on utilise habituellement les halogénures d'iridium. L'halogène est plus particulièrement choisi parmi le chlore, le brome ou l'iode, ce dernier étant préféré. Ainsi les composés comme IrI₃, IrBr₃, IrCl₃, IrI₃.4H₂O, IrI₄, IrBr₃.4H₂O peuvent être utilisés dans le procédé selon l'invention.

Les oxydes choisis parmi IrO₂, Ir₂O₃. xH₂O peuvent de même être convenablement mis en oeuvre dans le procédé selon l'invention.

En ce qui concerne les complexes de coordination solubles de l'iridium, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles d'iridium dont les ligands sont choisis parmi des composés organo phosphorés ou organo azotés, par exemple.

En tant que complexes de coordination connus de l'homme du métier, convenant particulièrement à la mise en oeuvre de l'invention, on peut citer sans intention de se limiter les composés suivants : Ir₄(CO)₁₂, Ir(CO)₂I₂⁻Q⁺, Ir(CO)₂Br₂⁻Q⁺, Ir(CO)₂Cl₂⁻Q⁺ ; formules dans lesquelles Q peut représenter notamment l'hydrogène, les groupes NR₄, PR₄, avec R choisi parmi l'hydrogène et/ou un radical hydrocarboné.

Ces catalyseurs peuvent être obtenus par toute méthode connue de l'homme du métier. Ainsi, on pourra se reporter aux brevets EP 657 386 et EP 737 103 pour la préparation de solutions catalytiques à base d'iridium convenant à la réalisation de la présente invention.

Comme indiqué précédemment, la réaction selon l'invention peut être mise en oeuvre avec un système catalytique comprenant, outre le platine, l'iridium seul mais aussi l'iridium et le rhodium.

Généralement, les composés à base de rhodium et d'iridium utilisés sont choisis parmi les complexes de coordination de ces métaux, solubles dans le milieu, dans les conditions de réaction. Plus particulièrement on utilise des complexes de coordination dont les ligands sont d'une part le monoxyde de carbone et d'autre part un halogène, comme le chlore, le brome ou plus particulièrement l'iode. On peut, bien entendu, mettre en oeuvre des complexes solubles comprenant d'autres ligands que ceux mentionnés, comme notamment des ligands organo-phosphorés ou azotés. Cependant, d'une façon avantageuse, la présente invention ne nécessite pas la mise en oeuvre de complexes de rhodium et d'iridium de ce type.

Ainsi, à titre d'exemples de complexes de coordination utilisés plus particulièrement dans la présente invention, on peut mentionner notamment les complexes du type Ir₄(CO)₁₂, -Ir(CO)₂I₂⁻Q⁺, Ir(CO)₂Br₂⁻Q⁺, Rh₄(CO)₁₂, Rh(CO)₂I₂⁻Q⁺, Rh(CO)₂Br₂⁻Q⁺, ou encore des complexes à base des deux métaux comme Rh₃Ir(CO)₁₂, Rh₂Ir₂(CO)₁₂ ; formules dans lesquelles Q peut représenter notamment l'hydrogène, les groupes NR₄, PR₄, avec R choisi parmi l'hydrogène et/ou un radical hydrocarboné.

Peuvent de même être mis en oeuvre dans le procédé selon l'invention, les composés choisis parmi les sels de ces éléments comme notamment IrI₃, IrBr₃, IrC1₃, IrI₃.4H₂O, IrBr₃.4H₂O, RhI₃, RhBr₃, RhCl₃, RhI₃.4H₂O, RhBr₃.4H₂O, ou encore le rhodium et l'iridium à l'état métallique.

Il est à noter que la liste des composés à base de rhodium et d'iridium mentionnée ci-dessus ne peut être considérée comme exhaustive, et qu'à titre d'exemples supplémentaires de composés des deux métaux précités, on pourra se référer aux brevets US 3 769 329 et US 3 772 380, dont l'enseignement est inclus ici.

Généralement, les composés à base de rhodium, d'iridium et de platine utilisés sont choisis parmi les complexes de coordination de ces métaux, solubles dans le milieu, dans les conditions de réaction. Plus particulièrement, on utilise des complexes de coordination dont les ligands sont d'une part le monoxyde de carbone et d'autre part un halogène, comme le chlore, le brome ou plus particulièrement l'iode. On peut, bien entendu, mettre en oeuvre des complexes solubles comprenant d'autres ligands que ceux mentionnés, comme notamment des ligands organo-phosphorés ou azotés. On peut mentionner également des complexes de deux ou trois des métaux précédemment cités, et sans intention de se limiter, on peut citer [PtRh₅(CO)₁₅]⁻Q⁺, Q étant tel que défini ci-dessus.

Une caractéristique importante de l'invention réside dans le fait que le platine se trouve dans le milieu réactionnel en quantité suffisante et dans des proportions adéquates par rapport à l'iridium. La concentration en platine est au moins égale à 4 mmol/l de milieu réactionnel et, de plus, le rapport atomique de l'iridium ou de l'ensemble (iridium + rhodium) au platine est compris entre 2 et 5, lorsque l'on utilise des composés simples du platine. Lorsque l'on introduit le platine sous forme de complexe de coordination, la concentration en platine est de préférence au moins égale à 1 mmol/l de milieu réactionnel et le rapport atomique de l'iridium au platine est compris entre 1 et 5. Il a été, en effet, observé de manière totalement inattendue que de telles conditions permettaient une augmentation considérable de la vitesse de réaction. Dans ces conditions, et de façon remarquable, la stabilité du système catalytique est également nettement améliorée.

Outre les composés mentionnés ci-dessus, le système catalytique selon l'invention comprend un promoteur halogéné. Celui-ci peut se présenter sous la forme d'un halogène seul, ou en combinaison avec d'autres éléments tels que, par exemple, l'hydrogène, le radical méthyle ou acétyle.

L'halogène est en général choisi parmi le chlore, le brome ou l'iode. l'iode étant préféré.

A titre de composés halogénés susceptibles d'être également utilisés en tant que promoteurs, on peut citer l'iode, l'acide iodhydrique, l'iodure de méthyle, l'iodure d'acétyle.

De préférence, on utilisera l'iodure de méthyle en tant que promoteur halogéné.

Selon une variante de l'invention, le promoteur halogéné est introduit dans le mélange réactionnel, partiellement ou totalement, sous la forme d'un précurseur. Dans un tel cas, ledit précurseur se présente généralement sous la forme d'un composé susceptible de libérer dans le milieu réactionnel, le radical hydrocarboné du promoteur halogéné précité, sous l'action d'un halogène ou de l'acide halohydrique notamment, ces derniers composés étant présents dans le milieu ou bien introduits dans ce but.

A titre d'exemples non limitatifs de précurseurs convenables, on peut citer les composés choisis parmi le méthanol, le diméthyléther, l'acétate de méthyle ou le formiate de méthyle utilisés seuls ou en mélange.

La quantité de promoteur halogéné présent dans le mélange réactionnel est avantageusement inférieure ou égale à 20 %, rapportée au poids total dudit mélange. De préférence, la teneur en promoteur halogéné est inférieure ou égale à 15 %.

Il est à noter que si le promoteur précité est introduit partiellement ou totalement, sous la forme d'un précurseur, la quantité de précurseur ou de mélange promoteur/précurseur est telle qu'elle permette d'obtenir une quantité équivalente à celle mentionnée ci-dessus.

Le procédé de l'invention peut être mis en oeuvre en alimentant au réacteur du méthanol en tant que seul réactif dans le cas de la carbonylation seule. Il peut aussi être mis en oeuvre en alimentant le réacteur en formiate de méthyle dans le cas d'un procédé par isomérisation, ou encore en formiate de méthyle et en méthanol dans le cas d'un procédé mixte par isomérisation et carbonylation simultanées.

La réaction selon l'invention est effectuée en outre en présence d'eau. Le procédé selon l'invention permet d'obtenir une bonne productivité à de faibles teneurs en eau, sans perte de métal catalytique par précipitation.

Ainsi, le procédé, objet de l'invention, peut être réalisé dans une large gamme de concentrations en eau dans le milieu réactionnel, mais de préférence à une concentration inférieure ou égale à 14 %, rapportée au poids total dudit milieu. Plus particulièrement la teneur en eau dans le milieu réactionnel est inférieure ou égale à 10 %.

Dans la première variante du procédé selon laquelle on effectue la carbonylation seule du méthanol, la teneur en eau est de préférence comprise entre 2 % et 8 % en poids du milieu réactionnel.

Dans la seconde variante mettant en oeuvre l'isomérisation du formiate de méthyle, éventuellement simultanément avec la carbonylation du méthanol, la teneur en eau est inférieure à 5 % et de préférence inférieure à 2 % en poids du milieu réactionnel.

Le procédé selon l'invention peut être mis en oeuvre en présence d'iodures sous forme soluble dans le milieu réactionnel. Les iodures peuvent être introduits en tant que tels dans le milieu réactionnel mais aussi sous la forme de composés susceptibles de former des iodures solubles.

Par iodures, on entend des espèces ioniques, c'est-à-dire ne comprenant pas les iodures covalents (tels que notamment le promoteur halogéné) ni l'acide iodhydrique.

Ainsi, les iodures introduits dans ledit mélange, en tant que tels, sont choisis parmi les iodures minéraux ou organiques.

A titre d'iodures minéraux, on peut citer principalement les iodures de métaux alcalino-terreux ou alcalins, ces derniers étant préférés. On peut citer parmi ceux-ci l'iodure de potassium, l'iodure de lithium, l'iodure de sodium.

A titre d'iodures organiques, on peut citer les composés organiques, comprenant au moins un groupe organo-phosphoré et/ou au moins un groupe organo-azoté, réagissant avec des composés à base d'iode, pour donner des espèces ioniques renfermant cet halogène. A titre d'exemple, on peut mentionner les composés de formules Q⁺, I⁻, dans lesquelles Q représente les groupes NR₄, PR₄ avec R choisi parmi l'hydrogène et/ou un radical hydrocarboné.

A titre de composés susceptibles de former des iodures solubles dans le milieu réactionnel, on peut citer par exemple les carboxylates, les hydroxydes de métaux alcalins ou alcalino-terreux, tels que l'acétate de lithium, la potasse, la soude notamment.

En outre, il est à noter que les iodures peuvent avoir d'autres origines que celles indiquées ci-dessus.

Ainsi, ces composés peuvent provenir d'impuretés comme les métaux alcalins ou alcalino-terreux, impuretés présentes dans les matières premières employées pour préparer la solution catalytique.

Les iodures peuvent de même provenir des métaux de corrosion apparaissant pendant la réaction. Il est préférable de maintenir le seuil de concentration de ces métaux relativement faible, de l'ordre de quelques centaines de parties par million, car ils ont notamment pour effet de favoriser la réaction de gaz à l'eau et contribuent à augmenter le rapport atomique iodures/iridium.

Il est possible d'introduire dans le milieu réactionnel une quantité d'iodures particulière selon la quantité d'iridium présent dans le milieu. Ainsi, cette quantité d'iodures introduite est telle que le rapport atomique iodures introduits/iridium (exprimé en mole/mole) est inférieur à 10 et maintenu dans cette gamme pendant la réaction.

Selon un mode de réalisation préféré de l'invention, le rapport atomique iodures/iridium est maintenu inférieur à 3. Plus particulièrement, ce rapport est inférieur à 1,5.

On a trouvé que l'ajout de telles quantités d'iodures permet d'améliorer la stabilité du catalyseur et de conserver une productivité importante au procédé.

Par conséquent, la présente invention est plus particulièrement destinée à être mise en oeuvre en continu et les conditions stables de fonctionnement du procédé correspondent à la composition et aux proportions indiquées.

Plus particulièrement, en ce qui concerne les iodures solubles, le maintien du rapport atomique iodures solubles/iridium peut être effectué en traitant un mélange comprenant au moins le composé de l'iridium, avec une résine échangeuse d'ions puis en ajoutant des iodures sous forme soluble dans une quantité telle que ledit rapport soit inférieur à 10.

Outre les composés et réactifs précédemment mentionnés, la réaction selon l'invention est avantageusement réalisée en présence d'esters.

De préférence, l'ester utilisé est l'acétate de méthyle et/ou le formiate de méthyle, utilisés en tant que tels ou sous forme masquée.

Selon un mode de réalisation de l'invention, la teneur en ester est avantageusement inférieure ou égale à 40 % en poids, rapportée au poids du mélange réactionnel. Plus particulièrement cette teneur reste inférieure ou égale à 30 %.

Enfin la réaction est effectuée en présence d'un solvant. Avantageusement, le solvant mis en oeuvre dans le procédé selon l'invention est l'acide acétique ou l'acide formique. Bien entendu, d'autres solvants peuvent être utilisés, comme notamment les composés inertes vis-à-vis du mélange réactionnel et présentant un point d'ébullition supérieur à celui de l'acide formé.

La réaction est en général mise en oeuvre à une température comprise entre 150 et 250°C. Plus particulièrement la température de réaction est comprise entre 175 et 210°C. De préférence, elle est comprise entre 175 et 200°C.

La pression totale sous laquelle est conduite la réaction est en général supérieure à la pression atmosphérique. Plus particulièrement elle est avantageusement inférieure à 200.10⁵ Pa et de préférence inférieure ou égale à 50.10⁵ Pa. Dans le cas d'un procédé selon l'invention mettant en oeuvre l'isomérisation du formiate de méthyle, éventuellement simultanément avec la carbonylation du méthanol, la pression partielle de CO sera avantageusement, comme indiqué précédemment, comprise entre 0,1.10⁵ Pa et 25.10⁵ Pa. Les pressions sont exprimées en pascals absolus et sont mesurées à chaud, c'est-à-dire dans les conditions de température de la réaction.

Le procédé selon l'invention est, de préférence, mis en oeuvre en présence d'une teneur en métaux de corrosion inférieure à quelques centaines de ppm, de préférence inférieure à 200 ppm. Les métaux de corrosion sont notamment le fer, le nickel, le chrome, le molybdène et le zirconium. La teneur en métaux de corrosion dans le mélange réactionnel est maintenue par les méthodes connues de l'homme du métier, comme par exemple la précipitation sélective, l'extraction liquide-liquide, le passage sur des résines échangeuses d'ions.

D'une façon générale, le procédé de l'invention est avantageusement mis en oeuvre en continu.

La réaction est mise en oeuvre dans des appareillages résistant à la corrosion créée par le milieu. Ainsi, le zirconium ou encore les alliages du type Hastelloy® C ou B conviennent particulièrement bien aux conditions de mise en oeuvre de la réaction.

Lors du démarrage de la réaction, les divers composants sont introduits dans un réacteur approprié, muni de moyens d'agitation pour assurer une bonne homogénéité du mélange réactionnel. Il est à noter que si le réacteur comprend de préférence des moyens d'agitation mécanique du mélange réactionnel, il n'est pas exclu d'opérer sans de tels moyens, l'homogénéisation du mélange pouvant être réalisée par l'introduction du monoxyde de carbone dans le réacteur.

Il est à noter que la réaction pourrait être convenablement mise en oeuvre dans un réacteur de type piston.

La combinaison de plusieurs réacteurs de type agité et piston est bien entendu envisageable.

L'introduction de monoxyde de carbone peut avoir lieu directement dans le réacteur où a lieu la réaction selon l'invention, mais aussi dans la zone de recyclage qui sera décrite ci-après.

Le mélange réactionnel en sortie du réacteur est traité de manière appropriée pour séparer les produits du mélange réactionnel comprenant notamment le catalyseur.

A ce titre, et dans le cas de mise en oeuvre de la réaction en continu, on peut employer par exemple une technique classique consistant à détendre le mélange de manière à provoquer une vaporisation partielle de ce dernier. Cette opération peut être mise en oeuvre grâce à une vanne permettant de détendre le mélange, ce dernier étant introduit dans un séparateur (dit séparateur flash). L'opération peut avoir lieu avec ou, de préférence, sans apport de chaleur, c'est-à-dire dans des conditions adiabatiques.

La partie non vaporisée, comprenant notamment le catalyseur resté en solution, est recyclée de manière avantageuse au réacteur, classiquement au moyen d'une pompe.

La partie vaporisée, comprenant en outre l'acide acétique et/ou l'acétate de méthyle produits, est ensuite envoyée dans une zone de purification, comprenant, de manière habituelle, diverses colonnes de distillation.

Enfin, le procédé selon l'invention peut être mis en oeuvre en intercalant entre le réacteur principal et la zone de vaporisation partielle, plus particulièrement en amont de la vanne de détente du mélange réactionnel, un réacteur supplémentaire dans lequel le monoxyde de carbone présent à l'état dissous et/ou entraîné sera consommé, entièrement ou partiellement.

Les conditions préférées de la mise en oeuvre d'un tel procédé peuvent être directement obtenues par l'homme du métier en se référant au brevet FR 2 750 984 mais en ajoutant, selon l'invention, du platine à l'iridium.

### EXEMPLES

### I - EXEMPLES DE CARBONYLATION DE MELANGES ACETATE DE METHYLE + METHANOL EN PRESENCE D'IODURE DE PLATINE II

### Exemples comparatifs A, B, C, D, E et exemples 1 et 2 selon l'invention

On a réalisé une série d'essais en tout point identiques entre eux, à l'exception de la nature et de la composition du système catalytique. Les conditions opératoires de ces essais sont détaillées dans l'essai comparatif A ci-dessous et le tableau n°1.

Les résultats obtenus dans les différents essais sont rassemblés dans le tableau n°2 ci-après dans lequel on a donné :
- dans la colonne intitulée V_{carb}, les vitesses de carbonylation calculées par rapport à la consommation de CO mesurée au réacteur après un temps de réaction de 10 minutes (TR = 10) correspondant à la quantité d'acide acétique formé par carbonylation pendant cette période. V_{carb} est exprimée en mole/l.h,
- dans la colonne intitulée TOF (TOF étant l'abréviation de l'expression anglaise Turnover Frequency), le rapport de la vitesse à la concentration en métal total. Le TOF est exprimé en h⁻¹.

### Essai comparatif A : Réaction de carbonylation en présence d'iridium seul

Tout d'abord, la solution catalytique est préparée comme suit :

On introduit dans un autoclave de 100 ml en HASTELLOY® B2,
- 0,454 g d'iodure d'iridium,
- 10 g d'acide acétique,
- 1 g d'eau.

L'autoclave est ensuite pressurisé sous 6 bars absolus de monoxyde de carbone à température ambiante.

On porte la température à 190°C.

La durée de la préparation de la solution catalytique est de 25 minutes.

La réaction de carbonylation est mise en oeuvre comme suit :

Dans l'autoclave, on injecte sous pression de CO, de l'acide acétique, de l'iodure de méthyle, de l'eau, du méthanol et de l'acétate de méthyle.

La composition initiale du mélange réactionnel est la suivante (en poids) :

| | |
|---|---|
| eau | 6,4% |
| acétate de méthyle | 30 % |
| iodure de méthyle | 10 % |
| méthanol | 5,7 % |
| iridium | 1 943 ppm |
| acide acétique | complément à 100 %. |

La pression totale est maintenue constante à 30 bars absolus par injection de monoxyde de carbone.

La température est maintenue à 190°C ± 0,5°C.

Après réaction, la masse de liquide réactionnel est de 72 g.

La vitesse de carbonylation (V_{carb}) est de 11 mol/l/h.

Le TOF est de 1 030 h⁻¹.

Après réaction, on observe un léger dépôt métallique dans l'autoclave.

### Essais comparatifs B, C, D et E

### Essais selon l'invention n°1 et 2

Ils sont réalisés dans les mêmes conditions opératoires et de composition initiale du mélange réactionnel, excepté pour les catalyseurs dont le détail est donné dans le tableau n°1, les catalyseurs étant introduits sous forme d'iodure d'iridium et/ou d'iodure de rhodium et/ou d'iodure de platine II.

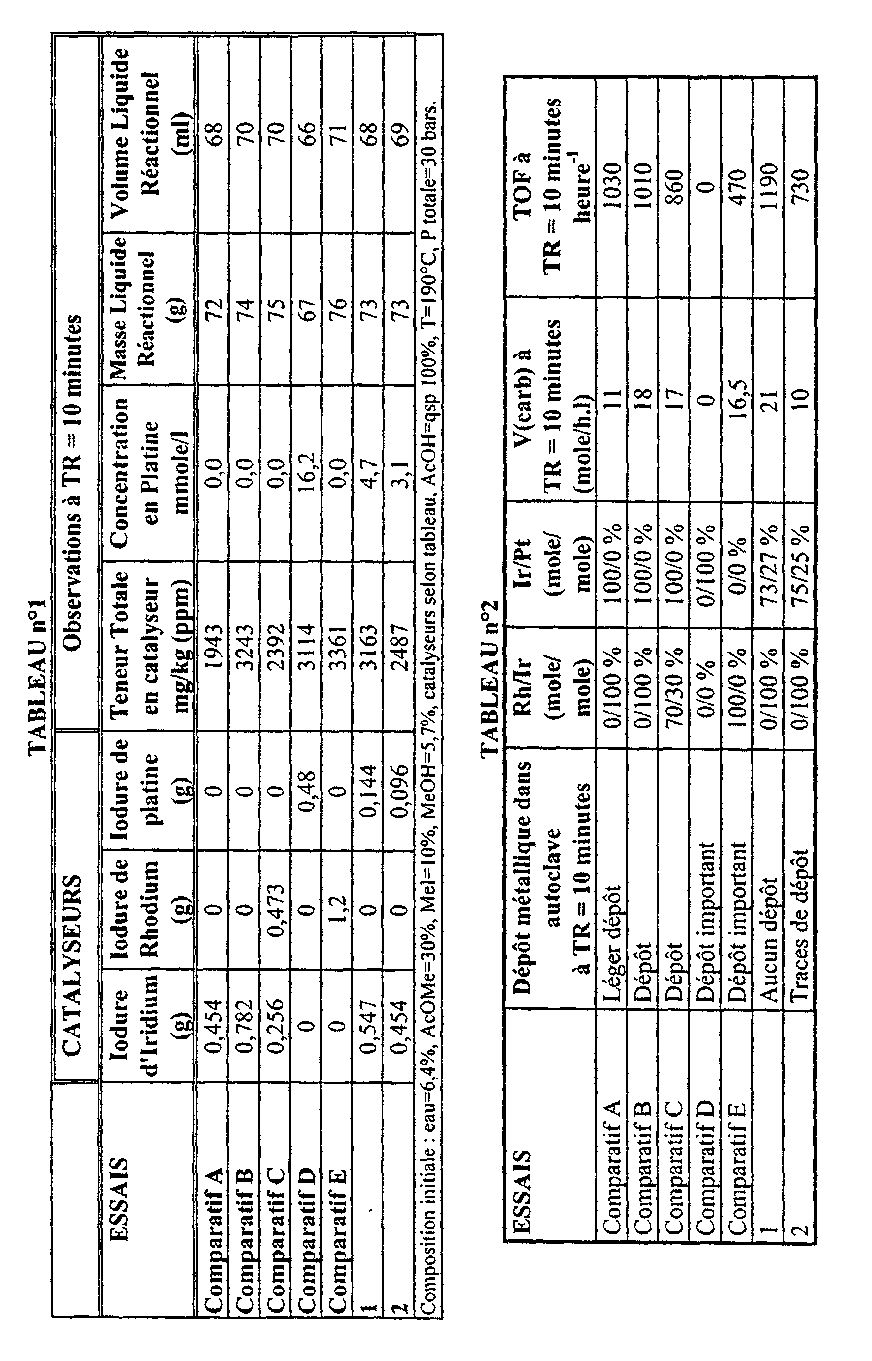

Comme cela ressort clairement du Tableau 2, l'ensemble des essais décrits ci-dessus met en évidence les points suivants :
* Le platine seul sous la forme iodure de platine II, PtI₂ n'a pas d'action catalytique pour la carbonylation du méthanol (essai D).
* Utilisé en quantité suffisante et dans des proportions adéquates par rapport à l'iridium (essai 1), le platine augmente l'activité catalytique de l'iridium pour la carbonylation du méthanol, comparativement au rhodium seul (essai E), à l'iridium seul (essais A et B), à l'iridium + rhodium (essai C).
* L'essai 2 qui n'a pas été réalisé dans les conditions optimales de l'invention (Pt = 3,1 mmol/l) montre toutefois une amélioration de la stabilité du catalyseur.
* La remarquable stabilité des catalyseurs dans l'essai selon l'invention n° 1 puisqu'aucun dépôt métallique d'iridium et/ou de platine n'est observé.

Le tableau n°2 met clairement en évidence l'amélioration de la vitesse de carbonylation pour une teneur suffisante en platine dans le système catalytique et un rapport atomique adéquat de l'iridium au platine.

### II - EXEMPLES DE CARBONYLATION D'ACETATE DE METHYLE EN PRESENCE DE TETRAIODODICARBONYLE DIPLATINE [PtI₂(CO)]₂

### Essais 3, 4, 5 et 6 selon l'invention

### Essais comparatifs F, G

On a réalisé une série d'essais en tout point identiques entre eux, à l'exception de la nature et de la composition du système catalytique. Les conditions opératoires de ces essais sont détaillées dans l'essai comparatif G ci-dessous et le tableau n°3.

Les résultats obtenus dans les différents essais sont rassemblés dans le tableau n°4 dans lequel on a donné :
- dans la colonne intitulée V_{carb}, les vitesses de carbonylation calculées par rapport à la consommation de CO mesurée au réacteur pour des concentrations données en acétate de méthyle - AcOMe - de 20 % et 15 % en poids dans le mélange réactionnel et correspondant à la quantité d'acide acétique formé par carbonylation V_{carb} exprimée en mole/l.h,
- dans la colonne intitulée TOF (TOF étant l'abréviation de l'expression anglaise Turnover Frequency), le rapport de la vitesse à la concentration en métal total, TOF exprimé en h⁻¹.

### Essai comparatif G : Réaction de carbonylation en présence d'iridium seul

Tout d'abord, la solution catalytique est préparée comme suit :

On introduit dans un autoclave de 100 ml en HASTELLOY® B2
- 0,4596 g d'iodure d'iridium,
- 10 g d'acide acétique,
- 1 g d'eau.

L'autoclave est ensuite pressurisé sous 6 bars absolus de monoxyde de carbone à température ambiante.

On porte la température à 190°C.

La durée de la préparation de la solution catalytique est de 25 minutes.

La réaction de carbonylation est mise en oeuvre comme suit :

Dans l'autoclave, on injecte sous pression de CO, de l'acide acétique, de l'iodure de méthyle, de l'eau et de l'acétate de méthyle.

La composition initiale du mélange réactionnel est la suivante (en poids) :

| | |
|---|---|
| eau | 6,4 % |
| acétate de méthyle | 30 % |
| iodure de méthyle | 10 % |
| méthanol | 0 % |
| iridium | 2 587 ppm |
| acide acétique | complément à 100 %. |

La pression totale est maintenue constante à 30 bars absolus par injection de monoxyde de carbone.

La température est maintenue à 190°C ± 0,5°C.

Après réaction, la masse de liquide réactionnel est de 52,3 g

La vitesse de carbonylation (V_{carb}) est de 16 mol/l/h et 14 mol/l/h, respectivement pour AcOMe 20 % et 15 % en poids.

Les TOF correspondants sont de 1 110 et 970 h⁻¹.

Après réaction, on observe un dépôt métallique dans l'autoclave.

### Essai comparatif F

### Essais selon l'invention n°3, 4, 5 et 6

Ils sont réalisés dans les mêmes conditions opératoires et de composition initiale du mélange réactionnel, excepté pour les catalyseurs dont le détail est donné dans le tableau n°3, les catalyseurs étant introduits sous forme d'iodure d'iridium et de tétraiododicarbonyle diplatine [PtI₂(CO)]₂.

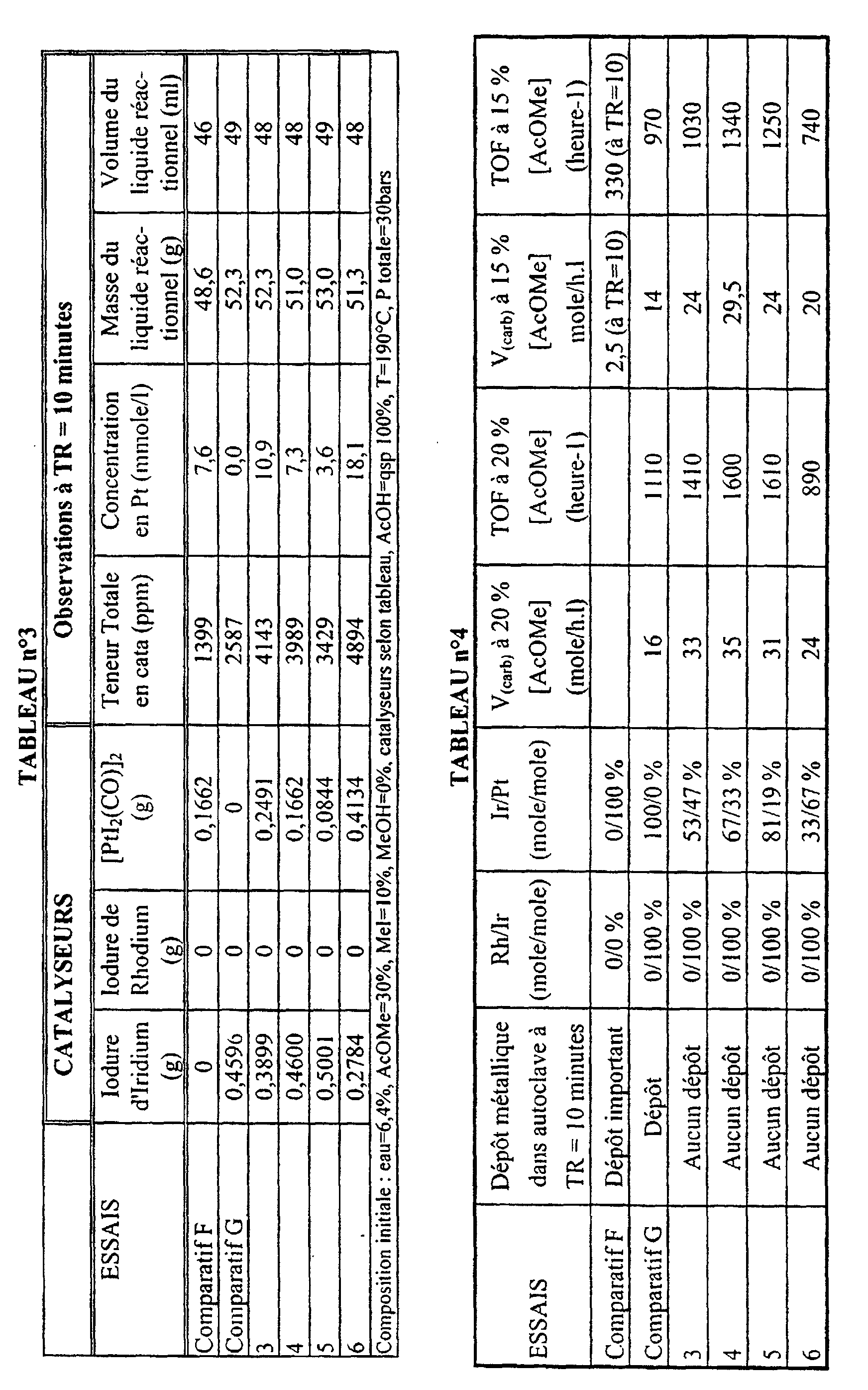

L'ensemble des essais décrits ci-dessus met en évidence les points suivants :
* Le platine seul employé sous forme de tétraiododicarbonyle diplatine, [PtI₂(CO)]₂, possède une action catalytique intéressante en termes de TOF (essai F = 330 h⁻¹) par rapport au platine seul sous forme non carbonylée (essai D = 0 h⁻¹).
* Utilisé en association, avec l'iridium (essais selon l'invention n°3, 4 et 5), en quantité suffisante et dans des proportions adéquates par rapport à l'iridium, le platine sous forme de tétraiododicarbonyle diplatine [PtI₂(CO)]₂ augmente l'activité de l'iridium pour la carbonylation de l'acétate de méthyle, comparativement à l'iridium seul (essai comparatif G).
* L'essai 6 qui n'a pas été réalisé dans les conditions optimales de l'invention (Ir/Pt *=* 0,5) montre toutefois une amélioration de la stabilité du catalyseur.
* La remarquable stabilité des catalyseurs dans les essais selon l'invention n°3, 4 et 5, puisque aucun dépôt métallique de platine et/ou d'iridium n'est observé.

Le tableau n°4 met clairement en évidence l'amélioration de la vitesse de carbonylation pour une teneur suffisante en platine dans le système catalytique et un rapport atomique adéquat de l'iridium au platine.

## Revendications

1. Procédé de préparation d'acide acétique et/ou d'acétate de méthyle en phase liquide par carbonylation du méthanol et/ou isomérisation du formiate de méthyle en présence d'eau, d'un solvant, d'un système catalytique homogène comprenant de l'iridium et un promoteur halogéné et de monoxyde de carbone, **caractérisé en ce que** ledit système catalytique comprend en outre du platine.

2. Procédé selon la revendication 1, **caractérisé en ce qu'**il s'agit d'un procédé de carbonylation du méthanol selon lequel on maintient une pression partielle en monoxyde de carbone comprise entre 0,1.10⁵ Pa et 200.10⁵ Pa pendant la réaction.

3. Procédé selon la revendication 1, **caractérisé en ce qu'**il s'agit d'un procédé d'isomérisation du formiate de méthyle selon lequel on maintient une pression partielle en monoxyde de carbone comprise entre 0,1.10⁵ Pa et 25.10⁵ Pa pendant la réaction.

4. Procédé selon la revendication 1, **caractérisé en ce qu'**il comprend simultanément une réaction de carbonylation de méthanol et d'isomérisation de formiate de méthyle et est réalisé sous une pression partielle en monoxyde de carbone comprise entre 0,1.10⁵ Pa et 25.10⁵ Pa pendant la réaction.

5. Procédé selon l'une des revendications 1 à 4, **caractérisé en ce que** le platine est introduit dans ledit système catalytique sous forme de platine à l'état métallique, d'un sel de platine ou d'un oxyde.

6. Procédé selon l'une des revendications 1 à 4, **caractérisé en ce que** le platine est introduit dans le système catalytique sous la forme d'un complexe de coordination, de préférence un complexe de coordination de ce métal avec des ligands choisis parmi le monoxyde de carbone ou une combinaison monoxyde de carbone/halogène ou des composés organoazotés ou organophosphorés.

7. Procédé selon la revendication 6, **caractérisé en ce que** ledit complexe est [PtI₂(CO)]₂.

8. Procédé selon la revendication 5, **caractérisé en ce que** l'on maintient le platine à une concentration d'au moins 4 mmoles/l de milieu réactionnel et un rapport atomique de l'iridium au platine compris entre 2 et 5.

9. Procédé selon la revendication 6 ou 7, **caractérisé en ce qu'**on maintient une teneur en platine d'au moins 1 mmole/l de milieu réactionnel et un rapport atomique de l'iridium au platine compris entre 1 et 5.

10. Procédé selon l'une des revendications 1 à 7, **caractérisé en ce que** ledit système catalytique contient en outre du rhodium.

11. Procédé selon la revendication 10, **caractérisé en ce que** le rapport atomique du rhodium à l'iridium est compris entre 0,01 et 99.

12. Procédé selon la revendication 10 ou 11, **caractérisé en ce que** le platine est introduit dans le système catalytique sous la forme de platine à l'état métallique, d'un sel ou d'un oxyde de platine et **en ce qu'**on maintient une teneur en platine d'au moins 4 mmoles/l de milieu réactionnel et un rapport atomique de l'ensemble (iridium + rhodium) au platine compris entre 2 et 5.

13. Procédé selon la revendication 10 ou 11, **caractérisé en ce que** le platine est introduit sous la forme d'un complexe de coordination tel que défini dans l'une des revendications 6 ou 7 et **en ce que** l'on maintient une teneur en platine d'au moins 1 mmole/l de milieu réactionnel et un rapport atomique de l'ensemble (iridium + rhodium) au platine compris entre 1 et 5.

14. Procédé selon l'une des revendications 1 à 13, **caractérisé en ce que** l'on met en oeuvre une concentration en iridium et, le cas échéant, en iridium et rhodium dans le milieu réactionnel comprise entre 0,1 et 100 mmol/l, de préférence entre 1 et 20 mmol/l.

15. Procédé selon l'une des revendications 1 à 14, **caractérisé en ce qu'**il est mis en oeuvre en présence d'une teneur en eau inférieure ou égale à 14 % en poids rapporté au poids total du milieu réactionnel et de préférence à une teneur inférieure ou égale à 10 % en poids.

16. Procédé selon l'une des revendications 1, 2 ou 5 à 15, **caractérisé en ce que** l'on effectue la carbonylation seule du méthanol et **en ce qu'**il est mis en oeuvre en présence d'une teneur en eau comprise entre 2 et 8 % en poids du milieu réactionnel.

17. Procédé selon la revendication 15, **caractérisé en ce qu'**il met en oeuvre une réaction d'isomérisation de formiate de méthyle et éventuellement une réaction simultanée de carbonylation de méthanol et **en ce qu'**il est mis en oeuvre en présence d'une teneur en eau inférieure à 5 % et de préférence inférieure à 2 % en poids du milieu réactionnel.

18. Procédé selon l'une des revendications 1 à 17, **caractérisé en ce que** ledit promoteur halogéné peut être l'halogène seul ou comprendre de l'hydrogène ou un radical méthyle ou acétyle.

19. Procédé selon la revendication 18, **caractérisé en ce que** ledit promoteur halogéné est l'iodure de méthyle.

20. Procédé selon l'une des revendications 1 à 19, **caractérisé en ce qu'**il est mis en oeuvre en présence d'une teneur en promoteur halogéné inférieure ou égale à 20 % en poids, rapporté au poids total du mélange réactionnel et, de préférence, inférieure à 15 %.

21. Procédé selon l'une des revendications 1 à 20, **caractérisé en ce qu'**il est mis en oeuvre en présence d'une teneur en ester inférieure à 40 % en poids, rapporté au poids total du mélange réactionnel et, de préférence, inférieure à 30 %.

22. Procédé selon l'une des revendications 1 à 21, **caractérisé en ce que** l'on introduit dans le milieu réactionnel des iodures solubles dans des proportions telles que le rapport atomique des iodures introduits dans le milieu réactionnel et de l'iridium soit maintenu inférieur à 10.

23. Procédé selon l'une quelconque des revendications 1 à 22, **caractérisé en ce qu'**il est mis en oeuvre en continu.

## Patentansprüche

1. Verfahren zur Herstellung von Essigsäure und/oder Methylacetat in flüssiger Phase durch Carbonylierung von Methanol und/oder Isomerisierung von Methylformiat in Gegenwart von Wasser, eines Lösungsmittels, eines homogenen katalytischen Systems, das Iridium und einen halogenierten Promotor umfasst, und von Kohlenmonoxid, **dadurch gekennzeichnet, dass** das genannte katalytische System außerdem Platin umfasst.

2. Verfahren nach Anspruch 1, **dadurch gekennzeichnet, dass** es sich dabei um ein Verfahren zur Carbonylierung von Methanol handelt, bei dem man während der Reaktion einen Kohlenmonoxid-Partialdruck zwischen 0,1.10⁵ und 200.10⁵ Pa aufrechterhält.

3. Verfahren nach Anspruch 1, **dadurch gekennzeichnet, dass** es sich dabei um ein Verfahren zur Isomerisierung von Methylformiat handelt, bei dem man während der Reaktion einen Kohlenmonoxid-Partialdruck zwischen 0_{,}1.10⁵ und 25.10⁵ Pa aufrechterhält

4. Verfahren nach Anspruch 1, **dadurch gekennzeichnet, dass** es umfasst die gleichzeitige Durchführung einer Reaktion zur Carbonylierung von Methanol und zur Isomerisierung von Methylformiat und dass es unter einem Kohlenmonoxid-Partialdruck zwischen 0,1.10⁵ und 25.10⁵ Pa während der Reaktion durchgeführt wird.

5. Verfahren nach einem der Ansprüche 1 bis 4, **dadurch gekennzeichnet, dass** das Platin in Form von metallischem Platin, in Form eines Platinsalzes oder eines Platinoxids in das katalytische System eingeführt wird.

6. Verfahren nach einem der Ansprüche 1 bis 4, **dadurch gekennzeichnet, dass** das Platin in Form eines Koordinationskomplexes, vorzugsweise eines Koordinationskomplexes dieses Metalls mit Liganden, ausgewählt aus Kohlenmonoxid oder einer Kombination von Kohlenmonoxid und Halogen oder Organostickstoff-Verbindungen oder Organophosphor-Verbindungen, in das katalytische System eingeführt wird.

7. Verfahren nach Anspruch 6, **dadurch gekennzeichnet, dass** es sich bei dem genannten Komplex um [PtI₂(CO)]₂ handelt

8. Verfahren nach Anspruch 5, **dadurch gekennzeichnet, dass** man das Platin bei einer Konzentration von mindestens 4 mmol/l Reaktionsmedium und bei einem Atomverhältnis von Iridium zu Platin zwischen 2 und 5 hält.

9. Verfahren nach Anspruch 6 oder 7, **dadurch gekennzeichnet, dass** man einen Platin-Gehalt von mindestens 1 mmol/l Reaktionsmedium und ein Atomverhältnis von Iridium zu Platin zwischen 1 und 5 aufrechterhält

10. Verfahren nach einem der Ansprüche 1 bis 7, **dadurch gekennzeichnet, dass** das katalytische System außerdem Rhodium enthält.

11. Verfahren nach Anspruch 10, **dadurch gekennzeichnet, dass** das Atomverhältnis von Rhodium zu lridium zwischen 0,01 und 99 liegt.

12. Verfahren nach Anspruch 10 oder 11, **dadurch gekennzeichnet, dass** das Platin in Form von metallischem Platin, in Form eines Salzes oder eines Oxids von Platin in das katalytische System eingeführt wird und dass man einen Platin-Gehalt von mindestens 4 mmol/l Reaktionsmedium und ein Atomverhältnis von der Gesamtmenge Iridium + Rhodium zu Platin zwischen 2 und 5 aufrechterhält.

13. Verfahren nach Anspruch 10 oder 11, **dadurch gekennzeichnet, dass** das Platin in Form eines Koordinationskomplexes, wie in einem der Ansprüche 6 oder 7 definiert, eingeführt wird und dass man einen Platin-Gehalt von mindestens 1 mmol/l Reaktionsmedium und ein Atomyerhältnis von der Gesamtmenge Iridium + Rhodium zu Platin zwischen 1 und 5 aufrechterhält.

14. Verfahren nach einem der Ansprüche 1 bis 13, **dadurch gekennzeichnet, dass** man Iridium und gegebenenfalls Iridium + Rhodium in einer Konzentration in dem Reaktionsmedium zwischen 0,1 und 100 mmol/l, vorzugsweise zwischen 1 und 20 mmol/l, verwendet.

15. Verfahren nach einem der Ansprüche 1 bis 14, **dadurch gekennzeichnet, dass** es es in Gegenwart eines Wassergehaltes von ≤ 14 Gew.-%, bezogen auf das Gesamtgewicht des Reaktionsmediums und vorzugsweise eines Wassergehaltes von ≤ 10 Gew.-% durchgeführt wird.

16. Verfahren nach einem der Ansprüche 1, 2 oder 5 bis 15, **dadurch gekennzeichnet, dass** man nur die Carbonylierung von Methanol durchführt und dass es in Gegenwart eines Wassergehaltes zwischen 2 und 8 Gew.-%, bezogen auf das Gesamtgewicht des Reaktionsmediums, durchgeführt wird.

17. Verfahren nach Anspruch 15, **dadurch gekennzeichnet, dass** man eine Reaktion zur Isomerisierung von Methylformiat und gegebenenfalls eine gleichzeitige Reaktion zur Carbonylierung von Methanol durchführt und dass es in Gegenwart eines Wassergehaltes von < 5 Gew.-%, vorzugsweise von < 2 Gew.-%, bezogen auf das Gesamtgewicht des Reaktionsmediums, durchgeführt wird.

18. Verfahren nach einem der Ansprüche 1 bis 17, **dadurch gekennzeichnet, dass** der genannte halogenierte Promotor nur Halogen sein kann oder Wasserstoff oder einen Methyl- oder Acetyl-Rest umfassen kann.

19. Verfahren nach Anspruch 18, **dadurch gekennzeichnet, dass** der genannte halogenierte Promotor Methyliodid ist.

20. Verfahren nach einem der Ansprüche 1 bis 19, **dadurch gekennzeichnet, dass** es in Gegenwart eines Gehaltes an dem halogenierten Promotor von ≤ 20 Gew.-%, bezogen auf das Gesamtgewicht der Reaktionsmischung, und vorzugsweise von < 15 Gew.-% durchgeführt wird.

21. Verfahren nach einem der Ansprüche 1 bis 20, **dadurch gekennzeichnet, dass** es in Gegenwart eines Ester-Gehaltes von < 40 Gew.-%, bezogen auf das Gesamtgewicht der Reaktionsmischung, und vorzugsweise von < 30 Gew.-% durchgeführt wird.

22. Verfahren nach einem der Ansprüche 1 bis 21, **dadurch gekennzeichnet, dass** man in das Reaktionsmedium lösliche Iodide in solchen Mengenanteilen einführt, dass das Atomverhältnis zwischen den in das Reaktionsmedium eingeführten Iodiden und dem Iridium bei < 10 gehalten wird.

23. Verfahren nach einem der Ansprüche 1 bis 22, **dadurch gekennzeichnet, dass** es kontinuierlich durchgeführt wird.

## Claims

1. A process for the preparation of acetic acid and/or methyl acetate in the liquid phase by the carbonylation of methanol and/or the isomerization of methyl formate in the presence of water, a solvent, a homogeneous catalyst system comprising iridium and a halogen-containing promoter, and carbon monoxide, **characterized in that** said catalyst system also comprises platinum.

2. The process according to claim 1 **characterized in that** it is a process for the carbonylation of methanol wherein a carbon monoxide partial pressure of between 0.1.10⁵ Pa and 200.10⁵ Pa is maintained throughout the reaction.

3. The process according to claim 1 **characterized in that** it is a process for the isomerization of methyl formate wherein a carbon monoxide partial pressure of between 0,1.10⁵ Pa and 25.10⁵ Pa is maintained throughout the reaction.

4. The process according to claim 1 **characterized in that** it comprises a methanol carbonylation reaction and a methyl formate isomerization reaction simultaneously and is carried out under a carbon monoxide partial pressure of between 0.1.10⁵ Pa and 25.10⁵ Pa throughout the reaction.

5. The process according to one of claims 1 to 4 **characterized in that** the platinum is introduced into said catalyst system in the form of platinum in the metallic state, a platinum salt or an oxide.

6. The process according to one of claims 1 to 4 **characterized in that** the platinum is introduced into the catalyst system in the form of a coordination complex, preferably a coordination complex of this metal with ligands selected from carbon monoxide, a carbon monoxide/halogen combination and organonitrogen and organophosphorus compounds.

7. The process according to claim 6 **characterized in that** said complex is [PtI₂(CO)]₂.

8. The process according to claim 5 **characterized in that** a platinum concentration of at least 4 mmol/l of reaction medium and an atomic ratio of iridium to platinum of between 2 and 5 are maintained.

9. The process according to claim 6 or 7 **characterized in that** a platinum content of at least 1 mmol/l of reaction medium and an atomic ratio of iridium to platinum of between 1 and 5 are maintained.

10. The process according to one of claims 1 to 7 **characterized in that** said catalyst system also contains rhodium.

11. The process according to claim 10 **characterized in that** the atomic ratio of rhodium to iridium is between 0.01 and 99.

12. The process according to claim 10 or 11 **characterized in that** the platinum is introduced into the catalyst system in the form of platinum in the metallic state, a platinum salt or a platinum oxide, and a platinum content of at least 4 mmol/l of reaction medium and an atomic ratio of (iridium + rhodium) to platinum of between 2 and 5 are maintained.

13. The process according to claim 10 or 11 **characterized in that** the platinum is introduced in the form of a coordination complex as defined in one of claims 6 or 7, and a platinum content of at least 1 mmol/l of reaction medium and an atomic ratio of (iridium + rhodium) to platinum of between 1 and 5 are maintained.

14. The process according to one of claims 1 to 13 **characterized in that** a concentration of iridium and, if appropriate, iridium and rhodium in the reaction medium of between 0.1 and 100 mmol/l, preferably of between 1 and 20 mmol/l, is used.

15. The process according to one of claims 1 to 14 **characterized in that** it is carried out in the presence of a water content less than or equal to 14% by weight, based on the total weight of the reaction medium, and preferably a content less than or equal to 10% by weight.

16. The process according to one of claims 1, 2 or 5 to 15 **characterized in that** only the carbonylation of methanol is carried out and this is done in the presence of a water content of between 2 and 8% by weight of the reaction medium.

17. The process according to claim 15 **characterized in that** it involves a methyl formate isomerization reaction and, if appropriate, a simultaneous methanol carbonylation reaction and is carried out in the presence of a water content of less than 5% and preferably of less than 2% by weight of the reaction medium.

18. The process according to one of claims 1 to 17 **characterized in that** said halogen-containing promoter can be the halogen by itself or can comprise hydrogen or a methyl or acetyl radical.

19. The process according to claim 18 **characterized in that** said halogen-containing promoter is methyl iodide.

20. The process according to one of claims 1 to 19 **characterized in that** it is carried out in the presence of a content of halogen-containing promoter less than or equal to 20% by weight, based on the total weight of the reaction mixture, and preferably less than 15%.

21. The process according to one of claims 1 to 20 **characterized in that** it is carried out in the presence of an ester content of less than 40% by weight, based on the total weight of the reaction mixture, and preferably of less than 30%.

22. The process according to one of claims 1 to 21 **characterized in that** iodides are introduced into the reaction medium in proportions such that the atomic ratio of soluble iodides introduced into the reaction medium to iridium is kept below 10.

23. The process according to any one of claims 1 to 22 **characterized in that** it is carried out continuously.
